# EUROPEAN PATENT APPLICATION

(11) **EP 4 361 257 A1**
(43) Date of publication of application: **01.05.2024**
(21) Application number: 22841271.4
(22) Date of filing: 08.07.2022
(51) Int. Cl.: C12N 7/01, C12N 7/04, A61K 48/00, A61P 35/00

(54) **ONCOLYTIC VIRUS AND USE THEREOF**

(30) Priority: 14.07.2021 CN 202110798531
(71) Applicant: Joint Biosciences (SH) Ltd., Pudong New Area Shanghai 201318 (CN)
(72) Inventor: ZHOU, Guoqing, Shanghai 201318 (CN); ZHANG, Fan, Shanghai 201318 (CN)
(74) Representative: Ullrich & Naumann PartG mbB
(86) International application number: PCT/CN2022/104525
(87) International publication number: WO 2023/284635

(57) **Abstract**

Provided is an oncolytic virus, including an M protein and a G protein. The M protein includes amino acid substitutions at positions 51, 221, and 226 compared with an amino acid sequence as shown in SEQ ID NO: 1. The G protein includes at least one amino acid substitution compared with an amino acid sequence as shown in SEQ ID NO: 2. Further provided are an expression vector of the oncolytic virus, a virus production cell for producing the oncolytic virus, and a pharmaceutical composition comprising the oncolytic virus, and a method for preparing the oncolytic virus, the expression vector of the oncolytic virus, the virus production cell, and/or the pharmaceutical composition, and an use thereof.

## Description

### TECHNICAL FIELD

The present application relates to a field of bio-medicine, and, particularly, to an oncolytic virus and an use thereof.

### BACKGROUND

The oncolytic virus is a tumor-killing virus with an ability to replicate and has been accepted by general public as an important branch of tumor immunotherapy. The oncolytic virus can specifically target and infect a tumor cell, for example, by exploiting an inactivation or a defect of a tumor suppressor gene in the tumor cell to selectively infect the tumor cell; after infecting the tumor cell, the oncolytic virus will replicate in large numbers within the tumor cell and eventually destroy the tumor cell, thereby killing the tumor cell. At the same time, the oncolytic virus can further provide an immune stimulation signal necessary to improve a host's own anti-cancer response, thereby attracting more immune cells to continue killing a residual tumor cell.

Although the oncolytic virus has a good application prospect in tumor immunotherapy, a wild-type oncolytic virus often causes an inflammation of a body's nervous system and other problems. A large pathogenic risk exists are in a process of using the wild-type virus to infect the tumor cell. Therefore, in order to further promote a clinical application of the oncolytic virus, the wild-type oncolytic virus needs to be engineered to obtain an attenuated oncolytic virus. The clinical application of the attenuated oncolytic virus can reduce a risk of the oncolytic virus and improve a safety of the oncolytic virus.

However, in a process of engineering the oncolytic virus, if only the wild-type oncolytic virus is randomly genetically engineered, although a toxicity of the wild-type oncolytic virus can be reduced, an engineered oncolytic virus may have a poor cure rate, or even the engineered oncolytic virus cannot be packaged, which is not conducive to promoting the clinical application of the oncolytic virus. Therefore, providing an engineered oncolytic virus with good safety performance and high cure rate has important scientific research value and application significance in a field of tumor immunotherapy.

### BRIEF SUMMARY

The present application provides an oncolytic virus and an use thereof. The oncolytic virus provided by the present application has good safety and cure rate.

In a first aspect, the present application provides an oncolytic virus, which adopts the following technical solution.

In the present application, the oncolytic virus includes an M protein and a G protein; the M protein includes amino acid substitutions at positions 51, 221, and 226 compared with an amino acid sequence as shown in SEQ ID NO: 1; the G protein includes at least one amino acid substitution compared with an amino acid sequence as shown in SEQ ID NO: 2.

In certain embodiments, the amino acid substitution of the M protein includes mutating of methionine at position 51 into arginine (M51R); and/or mutating of valine at position 221 into phenylalanine (V221F); and/or mutating of serine at position 226 into arginine (S226R).

In certain embodiments, the amino acid substitution of the M protein includes mutating of methionine at position 51 into arginine (M51R).

In certain embodiments, the amino acid substitution of the M protein includes mutating of valine at position 221 into phenylalanine (V221F).

In certain embodiments, the amino acid substitution of the M protein includes mutating of serine at position 226 into arginine (S226R).

In certain embodiments, the amino acid substitutions of the M protein include M51R and V221F.

In certain embodiments, the amino acid substitutions of the M protein include M51R and S226R.

In certain embodiments, the amino acid substitutions of the M protein include M51R, V221F, and S226R.

In certain embodiments, the amino acid substitutions of the M protein include V221F and S226R.

In certain embodiments, the M protein includes an amino acid sequence as shown in SEQ ID NO: 5.

In certain embodiments, the M protein includes an amino acid sequence as shown in SEQ ID NO: 3.

In certain embodiments, the M protein includes an amino acid sequence as shown in SEQ ID NO: 4.

In certain embodiments, compared with the amino acid sequence as shown in SEQ ID NO: 2, the G protein comprises amino acid substitutions at one or more of the following positions: position 453, position 471 and position 487.

In certain embodiments, compared with the amino acid sequence as shown in SEQ ID NO: 2, the G protein comprises amino acid substitutions at one or more of the following positions: position 53, position 141, position 172, position 217 position, position 232, position 331, position 371, position 436, position 438, position 453, position 471 and position 487.

In certain embodiments, the amino acid substitution of the G protein includes mutating of valine at position 53 into isoleucine (V53I); and/or mutating of alanine at position 141 into valine (A141V); and/or mutating of aspartic acid at position 172 into tyrosine (D172Y); and/or mutating of lysine at position 217 into glutamic acid (K217E); and/or mutating of aspartic acid at position 232 into glycine (D232G); and/or mutating of valine at position 331 into alanine (V331A ); and/or mutating of valine at position 371 into glutamic acid (V371E); and/or mutating of glycine at position 436 into aspartic acid (G436D); and/or mutating of valine at position 438 into serine (T438S); and/or mutating of phenylalanine at position 453 into leucine (F453L); and/or mutating of threonine at position 471 into isoleucine (T471I); and/or mutating of tyrosine at position 487 into histidine (Y487H).

In certain embodiments, the amino acid substitution of the G protein includes mutating of valine at position 53 into isoleucine (V53I).

In certain embodiments, the amino acid substitution of the G protein includes mutating of alanine at position 141 into valine (A141V).

In certain embodiments, the amino acid substitution of the G protein includes mutating of aspartic acid at position 172 into tyrosine (D172Y).

In certain embodiments, the amino acid substitution of the G protein includes mutating of lysine at position 217 into glutamic acid (K217E).

In certain embodiments, the amino acid substitution of the G protein includes mutating of aspartic acid at position 232 into glycine (D232G).

In certain embodiments, the amino acid substitution of the G protein includes mutating of valine at position 331 into alanine (V331A).

In certain embodiments, the amino acid substitution of the G protein includes mutating of valine at position 371 into glutamic acid (V371E).

In certain embodiments, the amino acid substitution of the G protein includes mutating of glycine at position 436 into aspartic acid (G436D).

In certain embodiments, the amino acid substitution of the G protein includes mutating of threonine at position 438 into serine (T438S).

In certain embodiments, the amino acid substitution of the G protein includes mutating of phenylalanine at position 453 into leucine (F453L).

In certain embodiments, the amino acid substitution of the G protein includes mutating of threonine at position 471 into isoleucine (T471I).

In certain embodiments, the amino acid substitution of the G protein includes mutating of tyrosine at position 487 into histidine (Y487H).

In certain embodiments, the amino acid substitutions of the G protein include T438S, F453L, T471I, and Y487H.

In certain embodiments, the amino acid substitutions of the G protein include V53I and A141V.

In certain embodiments, the amino acid substitutions of the G protein include V53I, A141V, and D172Y.

In certain embodiments, the amino acid substitutions of the G protein include V53I, A141V, D172Y, and K217E.

In certain embodiments, the amino acid substitutions of the G protein include V53I, A141V, D172Y, K217E, and D232G.

In certain embodiments, the amino acid substitutions of the G protein include V53I, A141V, D172Y, K217E, D232G, and V331A.

In certain embodiments, the amino acid substitutions of the G protein include V53I, A141V, D172Y, K217E, D232G, V331A, and V371E.

In certain embodiments, the amino acid substitutions of the G protein include V53I, A141V, D172Y, K217E, D232G, V331A, V371E, and G436D.

In certain embodiments, the amino acid substitutions of the G protein include V53I, A141V, D172Y, K217E, D232G, V331A, V371E, G436D, and T438S.

In certain embodiments, the amino acid substitutions of the G protein include V53I, A141V, D172Y, K217E, D232G, V331A, V371E, G436D, T438S, and F453L.

In certain embodiments, the amino acid substitutions of the G protein include V53I, A141V, D172Y, K217E, D232G, V331A, V371E, G436D, T438S, F453L, and T471I.

In certain embodiments, the amino acid substitutions of the G protein include V53I, A141V, D172Y, K217E, D232G, V331A, V371E, G436D, T438S, F453L, T471I, and Y487H.

In certain embodiments, the amino acid substitutions of the G protein include A141V, D172Y, K217E, D232G, V331A, V371E, G436D, T438S, F453L, T471I, and Y487H.

In certain embodiments, the amino acid substitutions of the G protein include D172Y, K217E, D232G, V331A, V371E, G436D, T438S, F453L, T471I, and Y487H.

In certain embodiments, the amino acid substitutions of the G protein include K217E, D232G, V331A, V371E, G436D, T438S, F453L, T471I, and Y487H.

In certain embodiments, the amino acid substitutions of the G protein include D232G, V331A, V371E, G436D, T438S, F453L, T471I, and Y487H.

In certain embodiments, the amino acid substitutions of the G protein include V331A, V371E, G436D, T438S, F453L, T471I, and Y487H.

In certain embodiments, the amino acid substitutions of the G protein include V371E, G436D, T438S, F453L, T471I, and Y487H.

In certain embodiments, the amino acid substitutions of the G protein include G436D, T438S, F453L, T471I, and Y487H.

In certain embodiments, the amino acid substitutions of the G protein include T438S, F453L, T471I, and Y487H.

In certain embodiments, the amino acid substitutions of the G protein include F453L, T471I, and Y487H.

In certain embodiments, the amino acid substitutions of the G protein include T471I and Y487H.

In certain embodiments, the amino acid substitutions of the G protein include V53I, A141V, D172Y, K217E, D232G, V331A, V371E, G436D, T438S, F453L, T471I, and Y487H.

In certain embodiments, the amino acid substitutions of the G protein are V53I, A141V, D172Y, K217E, D232G, V331A, V371E, G436D, T438S, F453L, T471I and Y487H.

In certain embodiments, the G protein comprises an amino acid sequence as shown in SEQ ID NO: 17.

In certain embodiments, the G protein comprises an amino acid sequence as shown in SEQ ID NO: 6.

In certain embodiments, the G protein comprises an amino acid sequence as shown in SEQ ID NO: 7.

In certain embodiments, the G protein comprises an amino acid sequence as shown in SEQ ID NO: 8.

In certain embodiments, the G protein comprises an amino acid sequence as shown in SEQ ID NO: 9.

In certain embodiments, the G protein comprises an amino acid sequence as shown in SEQ ID NO: 10.

In certain embodiments, the G protein comprises an amino acid sequence as shown in SEQ ID NO: 11.

In certain embodiments, the G protein comprises an amino acid sequence as shown in SEQ ID NO: 12.

In certain embodiments, the G protein comprises an amino acid sequence as shown in SEQ ID NO: 13.

In certain embodiments, the G protein comprises an amino acid sequence as shown in SEQ ID NO: 14.

In certain embodiments, the G protein comprises an amino acid sequence as shown in SEQ ID NO: 15.

In certain embodiments, the G protein comprises an amino acid sequence as shown in SEQ ID NO: 16.

In certain embodiments, the G protein comprises an amino acid sequence as shown in SEQ ID NO: 18.

In certain embodiments, the G protein comprises an amino acid sequence as shown in SEQ ID NO: 19.

In certain embodiments, the G protein comprises an amino acid sequence as shown in SEQ ID NO: 20.

In certain embodiments, the G protein comprises an amino acid sequence as shown in SEQ ID NO: 21.

In certain embodiments, the G protein comprises an amino acid sequence as shown in SEQ ID NO: 22.

In certain embodiments, the G protein comprises an amino acid sequence as shown in SEQ ID NO: 23.

In certain embodiments, the G protein comprises an amino acid sequence as shown in SEQ ID NO: 24.

In certain embodiments, the G protein comprises an amino acid sequence as shown in SEQ ID NO: 25.

In certain embodiments, the G protein comprises an amino acid sequence as shown in SEQ ID NO: 26.

In certain embodiments, the G protein comprises an amino acid sequence as shown in SEQ ID NO: 27.

In certain embodiments, the G protein comprises an amino acid sequence as shown in SEQ ID NO: 28.

In certain embodiments, the oncolytic virus is obtained by site-directed mutagenesis on a rhabdovirus basis.

In certain embodiments, the oncolytic virus is obtained by site-directed mutagenesis on a Vesicular Stomatitis Virus (abbreviated "VSV").

In certain embodiments, the oncolytic virus is obtained by site-directed mutagenesis on an Indiana MuddSummer subtype of the VSV.

In certain embodiments, the oncolytic virus includes or expresses an exogenous target protein.

In certain embodiments, the oncolytic virus includes a nucleic acid molecule, and the nucleic acid molecule includes a nucleic acid sequence encoding the M protein with the amino acid substitution and a nucleic acid sequence encoding the G protein with the amino acid substitution.

In certain embodiments, the oncolytic virus includes a nucleic acid sequence encoding the exogenous target protein.

In certain embodiments, the nucleic acid sequence encoding the exogenous target protein in the nucleic acid molecule is located between the nucleic acid sequence encoding the M protein with the amino acid substitution and the nucleic acid sequence encoding the G protein with the amino acid substitution.

In certain embodiments, the nucleic acid sequence encodes a N protein (nucleoprotein N), a L protein (large polymerase protein L), and a P protein (phosphoprotein P) of the oncolytic virus.

In a second aspect, the present application provides an expression vector of the oncolytic virus, which adopts the following technical solution.

An oncolytic virus expression vector capable of producing any one of the oncolytic viruses described in the present application.

In a third aspect, the present application provides a virus production cell, which adopts the following technical solution.

A virus production cell capable of producing any one of the oncolytic viruses described in the present application.

In a fourth aspect, this application provides a pharmaceutical composition, which adopts the following technical solution.

A pharmaceutical composition includes any one of the oncolytic viruses described in the present application, and optionally a pharmaceutically acceptable carrier.

In a fifth aspect, the present application provides a method for preparing the above-mentioned oncolytic viruse, expression vector of the oncolytic virus, virus production cell, and/or pharmaceutical composition.

In a sixth aspect, the present application provides an use of the above-mentioned oncolytic viruse, expression vector of the oncolytic virus, virus production cell, and/or pharmaceutical composition in preparing a medicine for preventing and/or treating a disease and/or a disorder.

In certain embodiments, the oncolytic virus, the expression vector of the oncolytic virus, the virus production cell and/or the pharmaceutical composition are used in a method for slow and sustained killing of an abnormally proliferative cell.

In certain embodiments, the disease and/or the disorder includes the abnormally proliferative cell selected from a tumor cell or a cell associated with tumor tissue; preferably, the tumor cell is a cancer cell; more preferably, the cancer cell is a metastatic cancer cell.

In certain embodiments, a tumor includes a solid tumor and/or a hematological tumor.

In summary, the present application has the following beneficial effects.

The oncolytic viruses provided in the present application all have better infective ability to LLC cells, MC38 cells and HeLa cells, and have better killing ability to LLC cells, MC38 cells and 4T1 cells in vitro, and are not easy to eliminate in LLC cells, MC38 cells and Hela cells. Moreover, the oncolytic viruses provided the present application all have poor infective ability and have poor killing ability to MEF cells in vitro, and are easy to eliminate in MEF cells. Therefore, the oncolytic virus in the present application can be better used for infecting and killing the tumor cells, the cancer cells and other cells, and is not easy to eliminate in the tumor cells, the cancer cells and other cells, further improving the cure rate of the oncolytic virus on the tumor cells, the cancer cells and other cells. At the same time, the oncolytic virus provided above can not damage normal cells and is easier to eliminate in the normal cells, further ensuring the safety of the normal cells.

### BRIEF DESCRIPTION OF DRAWINGS

Specific technical features of inventions related to the present application are shown in claims. Features and advantages of the inventions related to the present application can be better understood by referring to the exemplary embodiments and drawings described in detail below. A brief description of the drawings is as follows.
FIG. 1 shows a test result of an infective ability of the oncolytic virus prepared in the present application and a wild-type oncolytic virus to LLC cells.
FIG. 2 shows a test result of an infective ability of the oncolytic virus prepared in the present application and the wild-type oncolytic virus to MC38 cells.
FIG. 3 shows a test result of an infective ability of the oncolytic virus prepared in the present application and the wild-type oncolytic virus to HeLa cells.
FIG. 4 shows a test result of an infective ability of the oncolytic virus prepared in the present application and the wild-type oncolytic virus to MEF cells.
FIG. 5 shows a test result of a killing ability of the oncolytic virus prepared in the present application and the wild-type oncolytic virus to LLC cells in vitro.
FIG. 6 shows a test result of a killing ability of the oncolytic virus prepared in the present application and the wild-type oncolytic virus to MC38 cells in vitro.
FIG. 7 shows a test result of a killing ability of the oncolytic virus prepared in the present application and the wild-type oncolytic virus to 4T1 cells in vitro.
FIG. 8 shows a test result of a killing ability of the oncolytic virus prepared in the present application and the wild-type oncolytic virus to MEF cells in vitro.
FIG. 9 shows a test result of a elimination of the oncolytic virus prepared in the present application and the wild-type oncolytic virus in LLC cells.
FIG. 10 shows a test result of a elimination of the oncolytic virus prepared in the present application and the wild-type oncolytic virus in MC38 cells.
FIG. 11 shows a test result of a elimination of the oncolytic virus prepared in the present application and the wild-type oncolytic virus in HeLa cells.
FIG. 12 shows a test result of a elimination of the oncolytic virus prepared in the present application and the wild-type oncolytic virus in MEF cells.

In the above figures, 0 on an abscissa represents the wild-type oncolytic virus; 1 to 26 on the abscissa represent the oncolytic viruses prepared in Preparation Examples 1 to 26 respectively.

Log₁₀TCID50 on an ordinate represents a value of TCID50 calculated using Karber method. The larger of the value of Log₁₀TCID50, the better of the infective ability of the oncolytic virus to the cell; the smaller the value of Log₁₀TCID50, the poorer of the infective ability of the oncolytic virus to the cell.

OD₅₇₀ on the ordinate represents an OD value of the cell. The larger of the OD₅₇₀ value, the poorer of the killing ability of the oncolytic virus to the cell; the smaller the OD₅₇₀ value, the better of the killing ability of the oncolytic virus to the cell.

IFN-β level on the ordinate represents an expression of IFN-β gene. The larger of the value of IFN-β level, the easier of the oncolytic virus to eliminate in the cell; the smaller of the value of IFN-β level, the more difficult of the oncolytic virus to eliminate in the cell.

Those skilled in the art can easily perceive other aspects and advantages of the present application according to the following detailed description, which only shows and describes exemplary embodiments of the present application. As those skilled in the art will realize, contents of the present application enable those skilled in the art to modify the disclosed exemplary embodiments without departing from a spirit and a scope of inventions related to the present application. Accordingly, the description in drawings and specification of the present application is only illustrative, not restrictive.

### DETAILED DESCRIPTION

Embodiments of inventions related to the present application are illustrated with the following specific examples. Those skilled in the art can easily understand other advantages and effects of inventions related to the present application according to contents disclosed in the specification.

### Terms definition

In the present application, a term "oncolytic virus" generally refers to a virus capable of replicating in and killing tumor cells. Oncolytic viruses include, but are not limited to: VSV, poxvirus, herpes simplex virus, measles virus, Semliki Forest virus, poliovirus, reovirus, Seneca Valley virus, ECHO virus, Coxsackie virus, Newcastle disease virus and Malaba virus. In certain embodiments, the oncolytic virus is engineered to increase selectivity for tumor cells. In certain embodiments, the oncolytic virus is engineered to reduce its immunogenicity. In certain embodiments, the oncolytic virus described in the present application is a VSV. In certain embodiments, the VSV is a mutant of an Indiana MuddSummer subtype strain of the VSV. In certain embodiments, site-directed genetic mutagenesis of an M protein and a G protein of the VSV can be performed.

In certain embodiments, the oncolytic virus described in the present application can be an oncolytic virus that has been subjected to an engineering at gene level, such as engineered with one or more genetic modifications, thereby improving its tumor selectivity and/or to preferentially replicate in dividing cells. The engineering at gene level can be modification of genes involved in DNA replication, nucleic acid metabolism, host tropism, surface attachment, virulence, lysis and diffusion processes, or can be integration of an exogenous gene. The exogenous gene can include an exogenous immune regulatory gene, an exogenous screening gene, an exogenous reporter genes, etc. The oncolytic virus can also be an oncolytic virus that has been subjected to an engineering at amino acid level, such as an insertion, a deletion, or a substitution of one or more amino acids.

In the present application, a term "M protein" generally refers to a matrix protein of the VSV. The M protein is an important virulence factor of the VSV, and a protein in the VSV known to interfere with an innate immune response of mice. The term "M protein" further includes homologs, orthologs, variants, functional active segments and the like thereof. In the present application, the M protein of an Indiana MuddSummer subtype of a wild-type VSV may include an amino acid sequence as shown in SEQ ID NO:1. In the present application, the M protein of the oncolytic virus may include an amino acid sequence as shown in SEQ ID NO: 3-5.

In the present application, a term "G protein" generally refers to a glycoprotein of the VSV, also known as an envelope protein. The term "G protein" further includes homologs, orthologs, variants, functional active segments and the like thereof. In the present application, the G protein of the Indiana MuddSummer subtype of the wild-type VSV may include an amino acid sequence as shown in SEQ ID NO:2. In the present application, the G protein of the oncolytic virus may include an amino acid sequence as shown in SEQ ID NO: 6-28.

In the present application, a term "L protein" generally refers to a RNA polymerase protein of the VSV. A L gene of the VSV encodes a RNA poly E protein. The term "L protein" further includes homologs, orthologs, variants, functional active segments and the like thereof.

In the present application, a term "N protein" generally refers to a nucleocapsid protein of the VSV. The term "N protein" further includes homologs, orthologs, variants, functional active segments and the like thereof.

In the present application, a protein mutation site is generally expressed by "amino acid + amino acid site + (amino acid after mutation)". In the present application, the mutation includes but is not limited to an addition, a substitution, a deletion and/or a knocking out of the amino acid. For example, a term "M51R" generally refers to a mutation of methionine M at position 51 into arginine R.

In the present application, a term "amino acid substitution" generally refers to a replacement of one amino acid residue present in a parent sequence with another amino acid residue. Amino acids in the parent sequence may be substituted, for example, by chemical peptide synthesis or by recombinant methods known in the art. Thus, "substitution at position xx" generally refers to replacing an amino acid present at position xx with an alternative amino acid residue. In the present application, the amino acid substitution may include an amino acid mutation.

In the present application, a term "mutation/mutating" usually refers to changing a nucleotide or amino acid sequence of a wild-type molecule. Amino acid changes can include substitution, deletion, knocking out, insertion, addition and truncation of an amino acid, and processing or cutting of a protein.

In the present application, a term "nucleic acid molecule" generally refers to nucleotides of any length. In the present application, the term "nucleic acid molecule" may encode a protein contained in the oncolytic virus. In the present application, the nucleic acid molecule may include DNA and/or RNA. In certain cases, the RNA may include a single-stranded RNA (ssRNA) or a double-stranded RNA (dsRNA). The single-stranded RNA may include a sense RNA, an anti-sense RNA, or an ambiguous RNA.

In the present application, a term "expression vector" generally refers to a nucleic acid vector. Under appropriate conditions, the expression vector is usually able to express a target gene and/or a target protein. In certain embodiments of the present application, the expression vector includes a nucleic acid molecule for expressing one or more components of a virus (eg, an oncolytic virus). For example, the expression vector may include at least one viral genomic element and may be packaged into a virus or into a virion.

In the present application, a term "virus production cell" generally refers to a cell, a cell line or a cell culture that can or has contained the nucleic acid molecule or the expression vector described in the present application, or is capable of expressing the oncolytic virus described in the present application. The cell may include a progeny of a single host cell. The cell can be obtained by transfection in vitro using the expression vector described in the present application.

In the present application, a term "pharmaceutical composition" generally refers to a preparation that allows a biological activity of an active component to exit in an effective form, and does not include other components with an unacceptable toxicity to a subject to whom the preparation is to be administered. In certain embodiments, these preparations can include the active component of a medicine and a medical acceptable carrier. In certain embodiments, a medicine product includes the medicine product that is used by parenteral, transdermal, intracavitary, intraarterial, intrathecal and/or intranasal administration or is directly injected into tissues. The medicine product can be administered by different ways, such as intravenous, intraperitoneal, subcutaneous, intramuscular, topical or intradermal administration.

In the present application, a term "prevention/ preventing" generally refers to preventing an occurrence, an onset, a recurrence and/or a spread of a disease, or one or more symptoms of the disease by taking certain measures in advance. In the present application, a term "treatment/treating" generally refers to eliminating or alleviating the disease, or one or more symptoms related to the disease. In certain embodiments, "treatment/treating" generally refers to administrating one or more medicines to patients suffering from this disease to eliminate or alleviate the disease. In certain embodiments, "treatment/treating" can be administrating the pharmaceutical composition and /or the medicine product in the presence or absence of other medicines after onset of the symptoms of a specific disease. For example, the occurrence, a development, the recurrence and/or a matastasis of the tumor is prevented by using the pharmaceutical composition and /or the medicine product in the present application.

In the present application, a term "tumor" usually refers to any new pathological tissue proliferation. The tumor can be benign or malignant. In the present application, the tumor can be a solid tumor and/or a hematological tumor. For research, these tissues can be isolated from readily available resources by methods well known to those skilled in the art.

The present application provides an oncolytic virus based on a wild-type VSV, specifically based on a VSV Indiana strain, an Indiana MuddSummer subtype strain of the VSV, obtained by mutating a site on an amino acid sequence of an M protein and/or a G protein thereof. The amino acid sequence of the M protein is shown in SEQ ID NO: 1; the amino acid sequence of the G protein is shown in SEQ ID NO: 2. In the present application, the M protein can be engineered, and the G protein can also be engineered.

The VSV was modified as follows to obtain the oncolytic virus.

The oncolytic virus includes the M protein and the G protein; the M protein includes amino acid substitutions at positions 51, 221, and 226 compared with an amino acid sequence as shown in SEQ ID NO: 1; the G protein includes at least one amino acid substitution compared with an amino acid sequence as shown in SEQ ID NO: 2.

The amino acid substitution of the M protein includes mutating of methionine at position 51 into arginine (M51R); and/or mutating of valine at position 221 into phenylalanine (V221F); and/or mutating of serine at position 226 into arginine (S226R). For example, the M protein includes the amino acid sequence as shown in SEQ ID NO 5. The M protein includes an amino acid sequence as shown in SEQ ID NO: 5. In the present application, the M protein may further include amino acid substitutions at other positions.

In the present application, the amino acid substitution of the G protein includes mutating of valine at position 53 into isoleucine (V53I); and/or mutating of alanine at position 141 into valine (A141V); and/or mutating of aspartic acid at position 172 into tyrosine (D172Y); and/or mutating of lysine at position 217 into glutamic acid (K217E); and/or mutating of aspartic acid at position 232 into glycine (D232G); and/or mutating of valine at position 331 into alanine (V331A ); and/or mutating of valine at position 371 into glutamic acid (V371E); and/or mutating of glycine at position 436 into aspartic acid (G436D); and/or mutating of valine at position 438 into serine (T438S); and/or mutating of phenylalanine at position 453 into leucine (F453L); and/or mutating of threonine at position 471 into isoleucine (T471I); and/or mutating of tyrosine at position 487 into histidine (Y487H). The G protein includes an amino acid sequence as shown in SEQ ID NO: 17.

In the present application, the G protein may include amino acid mutations at position 53 and position 141.

In the present application, the G protein may include amino acid mutations at position 53, position 141 and position 172.

In the present application, the G protein may include amino acid mutations at position 53, position 141, position 172 and position 217.

In the present application, the G protein may include amino acid mutations at position 53, position 141, position 172, position 217 and position 232.

In the present application, the G protein may include amino acid mutations at position 53, position 141, position 172, position 217, position 232 and position 331.

In the present application, the G protein may include amino acid mutations at position53, position 141, position 172, position 217, position 232, position 331 and position 371.

In the present application, the G protein may include amino acid mutations at position 53, position 141, position 172, position 217, position 232, position 331, position 371 and position 436.

In the present application, the G protein may include amino acid mutations at position 53, position 141, position 172, position 217, position 232, position 331, position 371, position 436 and position 438.

In the present application, the G protein may include amino acid mutations at position 53, position 141, position 172, position 217, position 232, position 331, position 371, position 436, position 438 and position 453.

In the present application, the G protein may include amino acid mutations at position 53, position 141, position 172, position 217, position 232, position 331, position 371, position 436, position 438, position 453 and position 471.

In the present application, the G protein may include amino acid mutations at position 53, position 141, position 172, position 217, position 232, position 331, position 371, position 436, position 438, position 453, position 471 and position 487.

In the present application, the G protein may include amino acid mutations at position 141, position 172, position 217, position 232, position 331, position 371, position 436, position 438, position 453, position 471 and position 487.

In the present application, the G protein may include amino acid mutations at position 172, position 217, position 232, position 331, position 371, position 436, position 438, position 453, position 471 and position 487.

In the present application, the G protein may include amino acid mutations at position 217, position 232, position 331, position 371, position 436, position 438, position 453, position 471 and position 487.

In the present application, the G protein may include amino acid mutations at position 232, position 331, position 371, position 436, position 438, position 453, position 471 and position 487.

In the present application, the G protein may include amino acid mutations at position 331, position 371, position 436, position 438, position 453, position 471 and position 487.

In the present application, the G protein may include amino acid mutations at position 371, position 436, position 438, position 453, position 471 and position 487.

In the present application, the G protein may include amino acid mutations at position 436, position 438, position 453, position 471 and position 487.

In the present application, the G protein may include amino acid mutations at position 436, position 453, position 471 and position 487.

In the present application, the G protein may include amino acid mutations at position 453, position 471 and position 487.

In the present application, the G protein may include amino acid mutations at position 471 and position 487.

In the present application, the G protein may include an amino acid mutation at position 487.

In the present application, the G protein may further include amino acid substitutions at other positions.

In certain embodiments, the G protein includes at least one or more amino acid substitutions in a conserved region. For example, the conserved region may include amino acids at positions 437 to 461 of the G protein. In certain embodiments, the G protein includes at least one or more amino acid substitutions in a truncated region of a cytoplasmic domain. For example, the truncated region of the cytoplasmic domain may include amino acids at positions 483 to 511 of the G protein.

In the present application, the G protein may include at least amino acid substitutions at position 438, position 453, position 471 and position 487.

The oncolytic virus may also include a nucleic acid molecule and an exogenous target protein. The nucleic acid molecule may include a nucleic acid sequence encoding the M protein with the amino acid substitution and a nucleic acid sequence encoding the G protein with the amino acid substitution; the nucleic acid molecule may further include a nucleic acid sequence encoding the exogenous target protein. Further, the nucleic acid sequence encoding the exogenous target protein in the nucleic acid molecule is located between the nucleic acid sequence encoding the M protein with the amino acid substitution and the nucleic acid sequence encoding the G protein with the amino acid substitution. Furthermore, the nucleic acid molecule encodes a N protein (nucleoprotein N), a L protein (large polymerase protein L), and a P protein (phosphoprotein P) of the oncolytic virus.

In the present application, the oncolytic virus described in the present application can be obtained through a virus packaging process and a virus rescue process. Specifically, the process may include: infecting and inoculating a BSR-T7 cell with a poxvirus vTF7-3 expressing T7 RNA polymerase, and transfecting with lipofectamine using a expression plasmid and a backbone plasmid respectively cloning VSV N, VSV P, and VSV L genes to obtain a target oncolysis virus.

The present application further provides an expression vector of the oncolytic virus, a virus production cell and a pharmaceutical composition.

The expression vector of the oncolytic virus may include nucleic acid sequences encoding the M protein and G protein of the oncolytic virus. The expression vector of the oncolytic virus may further include nucleic acid sequences encoding the N protein, P protein and L protein of the oncolytic virus.

The virus production cell is capable of producing the oncolytic virus as described above. The virus production cell can include the BSR-T7 cell.

The pharmaceutical composition includes the oncolytic virus as described above, and optionally a pharmaceutically acceptable carrier.

In certain embodiments, the pharmaceutical composition may include a suitable formulation of one or more (pharmaceutically effective) adjuvants, stabilizers, excipients, diluents, solubilizers, surfactants, emulsifiers, and/or preservatives. An acceptable ingredient of the pharmaceutical composition is preferably non-toxic to a recipient at a dose and a concentration used. The pharmaceutical composition of the present application includes, but is not limited to, a liquid composition, a frozen composition and a lyophilized composition.

In certain embodiments, the pharmaceutically acceptable carrier may include any and all solvents, dispersion media, coatings, isotonic agents and absorption delaying agents that are compatible with pharmaceutical administration and are generally safe and non-toxic.

In certain embodiments, the pharmaceutical composition may include parenteral, transdermal, intraluminal, intraarterial, intrathecal and/or intranasal administration or direct injection into a tissue. For example, the pharmaceutical composition can be administered to a patient or subject by infusion or injection. In certain embodiments, the administration of the pharmaceutical composition can be performed by various means, such as intravenous, intraperitoneal, subcutaneous, intramuscular, topical or intradermal administration. In certain embodiments, the pharmaceutical composition can be administered uninterrupted. Uninterrupted (or continuous) administration can be achieved by a small pump system worn by the patient to measure a flow of a therapeutic agent into the patient, as described in WO2015/036583.

In addition, the present application further provides a method for preparing the oncolytic virus as described above, which may include a method for preparing the expression vector of the oncolytic virus, the virus production cell, and/or the pharmaceutical composition. Any method suitable for producing the oncolytic virus can be used to produce the oncolytic virus of the present application. For example, a poxvirus expressing T7 RNA polymerase can be added to a cell for transfection, a plasmid and a backbone plasmid expressing the N protein, L protein, and P protein of the oncolytic virus can be added for transfection, and the oncolytic virus of the present application can be obtained through a virus rescue process.

The present application further provides an use of the oncolytic virus, the expression vector of the oncolytic virus, the virus production cell and/or the pharmaceutical composition as described above in preparing a medicine for preventing and/or treating a disease and/or a disorder.

The oncolytic viruses provided in the present application all have better infective ability to LLC cells, MC38 cells and HeLa cells, especially the oncolytic virus numbered JBS104-JBS126. Based on the oncolytic virus numbered JBS103, a site-directed mutation of the amino acids on the G protein of the oncolytic virus further improved an infective ability of the oncolytic virus to LLC cells, MC38 cells and HeLa cells. At the same time, the oncolytic viruses prepared above have poor infective ability to normal MEF cells, indicating that the oncolytic viruses prepared in the present application can be better used to infect tumor cells, cancer cells and other cells without damaging normal cells, and has a broad application prospect.

The oncolytic viruses provided in the present application all have better killing ability to LLC cells, MC38 cells and 4T1 cells in vitro, especially the oncolytic virus numbered JBS104-JBS126. Based on the oncolytic virus numbered JBS103, a site-directed mutation of the amino acids on the G protein of the oncolytic virus further improved a killing ability of the oncolytic virus to LLC cells, MC38 cells and 4T1 cells in vitro. At the same time, the oncolytic viruses prepared above have almost no effect on MEF cells, indicating that the oncolytic viruses prepared in the present application can be better used to damage and kill abnormal cells such as tumor cells, cancer cells without damaging normal cells.

Oncolytic viruses numbered JBS101 and JBS102 are not easy to eliminate in LLC cells, MC38 cells and HeLa cells. In contrast, the oncolytic virus numbered JBS103 is easier to eliminate in LLC cells, MC38 cells and HeLa cells. Based on the oncolytic virus numbered JBS103, the amino acids on the G protein of the oncolytic viruses numbered JBS104 to JBS126 in the present application are subjected to site-directed mutations, so that the oncolytic viruses can be more difficult to eliminate in LLC cells, MC38 and HeLa cells, further ensuring that the oncolytic viruses can better infect and kill in LLC cells, MC38 cells and HeLa cells. At the same time, the oncolytic virus provided in the present application is easier to eliminate in MEF cells, further ensures the safety of MEF cells, thereby improving the safety of the oncolytic virus.

Not limited by any theories, the following examples are only used to illustrate the various technical solutions in the present application, not to limit the scope of the present application.

### Preparation Examples

### Preparation Examples 1 to 3

Preparation Examples 1 to 3 each provide an oncolytic virus that differs primarily lies in a site of the site-directed mutation of the amino acid on the M protein of the wild-type oncolytic virus. A construction method of the oncolytic virus corresponding to each Preparation Example was as follows.

### (1) Plasmid constructing

Using pRV-core plasmid (BioVector NTCC Plasmid Vector Cell Gene Collection Center) as a template, and different mutation sites described in Table 1 were introduced by PCR method. The pRV-core plasmid was subjected to PCR with primers carrying each mutation site, and PCR products were then subjected to 1% agarose gel electrophoresis, and then an extraction to cutted gels was performed by using a gel extraction kit to obtain the plasmids with different mutation sites of the M protein, thereby a constructed plasmid pRV-core Mut was obtained.

**Table 1 mutation situation of M protein of oncolytic virus in Preparation Examples 1 to 3**

| No. of Preparation Example | Number | Mutation Site and Amino Acid after Mutation | No. of Mutation Sites | Amino Acid Sequence |
|---|---|---|---|---|
| 1 | JBS101 | M51R | 1 | SEQ ID NO: 3 |
| 2 | JBS102 | M51R, V221F | 2 | SEQ ID NO: 4 |
| 3 | JBS103 | M51R, V221F, S226R | 3 | SEQ ID NO: 5 |

### (2)Virus rescue

The constructed plasmid pRV-core Mut was transfected into BSR-T7 cells (purchased from ATCC, American Type Culture Collection, also known as American model) by using a calcium phosphate transfection kit (Thermo Fisher Scientific) and cell transfection method.

Four plasmids were mixed according to a mass ratio of pRV-core Mut, pP, pN, and pL to 10:5:4:1, and a total weight of the plasmids was 5 µg. The plasmids were diluted by using 200 µL of opti-MEM medium (Thermo Fisher Scientific) , and 7.5 µL of transfection reagent Plus Reagent (Life Technologies) was added to obtain a transfection plasmid premix; wherein the pP was a plasmid carrying a phosphoprotein gene of a rhabdovirus, the pN was a plasmid carrying a nucleoprotein gene of the rhabdovirus), the pL was a plasmid carrying a polymerase protein gene of the rhabdovirus. Parent vectors corresponding to the three plasmids pN, pP, and pL were pCAGGS (purchased from ATCC).

10 µL of Lipofectamine LTX (Thermo Fisher Scientific) was diluted by using 200 µL of opti-MEM medium to obtain a LTX mixture.

A plasmid transfection was performed according to a method described in a user manual of lipofectamine LTX. After 6 hours, the BSR-T7 cells were washed twice by using PBS, and further inoculated into DMEM medium (Thermo Fisher Scientific) containing 10% of fetal calf serum and cultured for 3 days.

A cell supernatant obtained by culturing the BSR-T7 cells was transferred into Vero cells (Thermo Fisher Scientific), and the Vero cells were cultured for 3 days under the environmental condition of 37°C. A green fluorescence in cells was observed with a fluorescence microscope to determine the virus rescue. A rescued mutant baculovirus library was further passed through the Vero cells, and monoclonal virus strains were selected in an established plaque screening system.

### (3)M protein sequencing.

A viral genome RNA was extracted with a Trizol kit, and a reverse transcription reaction was performed with random primers, and then a reverse transcribed cDNA was subjected to PCR with primers designed for a gene sequence of the M protein.

Sequences of the primer were as follows.
5' -AAAAAAGTAACAGATATCAC-3 ';
5'-ACATTTTTCCAGTTTCCTTTTTGG-3'.

A product was extracted after 1% agarose gel electrophoresis and sent to a sequencing company for sequencing. The sequencing results were shown in Table 1.

### Preparation Examples 4 to 26

Preparation Examples 4 to 26 each provide an oncolytic virus that differs primarily lies in a site of the site-directed mutation of the amino acid on the M protein and the G protein of the wild-type oncolytic virus. The mutation sites of the M protein were the same as that in Preparation Example 3. A construction method of the oncolytic virus corresponding to each Preparation Example was the same as the construction method described in Preparation Examples 1 to 3, except that the follows.

In step (1) of the plasmid constructing, mutation sites described in Table 2 were introduced by PCR method.

Step (3) was G protein sequencing. A viral genome RNA was extracted with a Trizol kit, and a reverse transcription reaction was performed with random primers, and then a reverse transcribed cDNA was subjected to PCR with primers designed for a gene sequence of the G protein.

Sequences of the primer were as follows.
5'-CCATGGCCTGTTGCTCCACCAGCTT-3';
5' -AAGCTTTCAGCAGTGGCTCACAGCAG-3'.

A product was extracted after 1% agarose gel electrophoresis and sent to a sequencing company for sequencing. The sequencing results were shown in Table 2.

**Table 2 mutation situation of G protein of oncolytic virus in Preparation Examples 4 to 26**

| No. of Preparation Example | Number | Mutation Site and Amino Acid after Mutation | No. of Mutation Sites | Amino Acid Sequence |
|---|---|---|---|---|
| 4 | JBS104 | V53I | 1 | SEQ ID NO: 6 |
| 5 | JBS105 | V53I, A141V | 2 | SEQ ID NO: 7 |
| 6 | JBS106 | V53I, A141V, D172Y | 3 | SEQ ID NO: 8 |
| 7 | JBS107 | V53I, A141V, D172Y, K217E | 4 | SEQ ID NO: 9 |
| 8 | JBS108 | V53I, A141V, D172Y, K217E, D232G | 5 | SEQ ID NO: 10 |
| 9 | JBS109 | V53I, A141V, D172Y, K217E, D232G, V331A | 6 | SEQ ID NO: 11 |
| 10 | JBS110 | V53I, A141V, D172Y, K217E, D232G, V331A, | 7 | SEQ ID NO: 12 |
| | | V371E | | |
| 11 | JBS111 | V53I, A141V, D172Y, K217E, D232G, V331A, V371E, G436D | 8 | SEQ ID NO: 13 |
| 12 | JBS112 | V53I, A141V, D172Y, K217E, D232G, V331A, V371E, G436D, T438S | 9 | SEQ ID NO: 14 |
| 13 | JBS113 | V53I, A141V, D172Y, K217E, D232G, V331A, V371E, G436D, T438S, F453L | 10 | SEQ ID NO: 15 |
| 14 | JBS114 | V53I, A141V, D172Y, K217E, D232G, V331A, V371E, G436D, T438S, F453L, T471I | 11 | SEQ ID NO: 16 |
| 15 | JBS115 | V53I, A141V, D172Y, K217E, D232G, V331A, V371E, G436D, T438S, F453L, T471I, Y487H | 12 | SEQ ID NO 17 |
| 16 | JBS116 | A141V, D172Y, K217E, D232G, V331A, V371E, G436D, T438S, F453L, T471I, Y487H | 11 | SEQ ID NO: 18 |
| 17 | JBS117 | D172Y, K217E, D232G, V331A, V371E, G436D, T438S, F453L, T471I, Y487H | 10 | SEQ ID NO: 19 |
| 18 | JBS118 | K217E, D232G, V331A, V371E, G436D, T438S, F453L, T471I, Y487H | 9 | SEQ ID NO 20 |
| 19 | JBS119 | D232G, V331A, V371E, G436D, T438S, F453L, T471I, Y487H | 8 | SEQ ID NO: 21 |
| 20 | JBS120 | V331A, V371E, G436D, T438S, F453L, T471I, Y487H | 7 | SEQ ID NO: 22 |
| 21 | JBS121 | V371E, G436D, T438S, F453L, T471I, Y487H | 6 | SEQ ID NO: 23 |
| 22 | JBS122 | G436D, T438S, F453L, T471I, Y487H | 5 | SEQ ID NO: 24 |
| 23 | JBS123 | T438S, F453L, T471I, Y487H | 4 | SEQ ID NO: 25 |
| 24 | JBS124 | F453L, T471I, Y487H | 3 | SEQ ID NO: 26 |
| 25 | JBS125 | T471I, Y487H | 2 | SEQ ID NO: 27 |
| 26 | JBS126 | Y487H | 1 | SEQ ID NO: 28 |

### Preparation Example 27

This Preparation Example provides a packaging process for the oncolytic virus prepared in the above Preparation Examples 1 to 26. Specifically, the packaging process included the following steps.
1) BSR-T7 cells (purchased from ATCC) were infected and inoculated using poxvirus vTF7-3 expressing T7 RNA polymerase (BioVector NTCC Plasmid Vector Cell Gene Collection Center).

Specifically, steps were as follows. The BSR-T7 cells were plated on a 6-well plate, and a number of cells per well was controlled to reach 3×10⁵, and then the poxvirus vTF7-3 expressing T7 RNA polymerase was added after plating 14 to16 hours, and then an infection of the BSR-T7 cells with the poxvirus vTF7-3 was performed. After 6 hours of the infection, the BSR-T7 cells were washed once by using DPBS buffer (Thermo Fisher Scientific) for transfection.

### Transfection process

Specifically, the transfection process included the following steps. Four plasmids were mixed according to a mass ratio of pRV-core Mut, pP, pN, and pL to 10:5:4:1, and a total weight of the plasmids was 5 µg. The plasmids were diluted by using 200 µL of opti-MEM medium (Thermo Fisher Scientific) , and 7.5 µL of transfection reagent Plus Reagent (Life Technologies) was added to obtain a transfection plasmid premix; wherein the pP was a plasmid carrying a phosphoprotein gene of a rhabdovirus, the pN was a plasmid carrying a nucleoprotein gene of the rhabdovirus, the pL was a plasmid carrying a polymerase protein gene of the rhabdovirus. Parent vectors corresponding to the three plasmids pN, pP, and pL were pCAGGS (purchased from ATCC).

10 µL of Lipofectamine LTX (Thermo Fisher Scientific) was diluted by using 200 µL of opti-MEM medium to obtain a LTX mixture.

200 µL of the LTX mixture was mix with 200 µl of the transfection plasmid premix, and then incubated at room temperature for 15 minutes to obtain a LTX-DNA mixture.

The DPBS buffer in the 6-well plate in step 1) was replaced with Opti-MEM medium, and then the LTX-DNA mixture was dropwise added into the 6-well plate where the BSR-T7 cells were cultured, and the 6-well plate was gently shaken to uniformly distribute the LTX-DNA mixture in the 6-well plate. After 6 to 8 hours of the transfection, the transfection reagent was removed and 3 mL of fresh complete culture medium (Thermo Fisher Scientific) was added. After 72 hours, a cell supernatant of the BSR-T7 cells was obtained and filtered by using a 0.22 µm filter to obtain oncolytic virus drops corresponding to each of Preparation Examples 1 to 26.

### Examples

### Example 1

In this Example, an infective ability of the oncolytic viruses prepared in Preparation Examples 1 to 26 and the wild-type oncolytic virus to different cells were respectively detected. A detection method was a TCID50 detection method, that is, 200 pfu each of the oncolytic viruses prepared in Preparation Examples 1 to 26 and the wild-type oncolytic virus were added into a culture medium of different cells, and half of a Tissue culture infectious dose (TCID50) generated by each oncolytic virus was detected. The cells tested included: LLC cells (mouse non-small cell lung cancer cells), MC38 cells (mouse colon cancer cells), Hela cells (human cervical cancer cells), and MEF cells (human fibroblasts). Specifically, the detection method included the following steps.
(1) 3mL of Vero (LLC/MC38/Hela/MEF) cell suspension was added into each well of a 6-well culture plate to make a cell number reach 4×10⁵/well, 6 wells for each type of the Vero cells, and 2 wells for MEF cells as control. The 6-well culture plate was cultured for 16 hours under an environmental condition of 37°C and 5% of CO₂.
(2) 200 pfu of the oncolytic virus prepared in Preparation Example was added into each well of the 6-well culture plate. At 24 hours, the MEF cells and 100 µL of a supernate of each Vero cells were obtained. The MEF cells obtained were added into wells of a 96-well culture plate respectively to make the cell number of various cells reach 1×10⁴/mL, and the 96-well culture plate was cultured for 16 hours in the environmental condition of 37°C and 5% of CO₂.
(3) The supernate obtained in step (2) was diluted at a 10-fold dilution ratio serially in a 1.5m LEP tube, a total of 11 titers ranging from 10⁻¹ to 10⁻¹¹ were obtained. The diluted supernates were inoculated into the 96-well culture plate, one column (8 wells in total) for each dilution was inoculated, and 100 µL was inoculated in each well.
(4) A fluorescence of cells in each well was observed after 48 hours, if there was the fluorescence, then the well was marked with being infected. The TCID50 was calculated using Karber method.

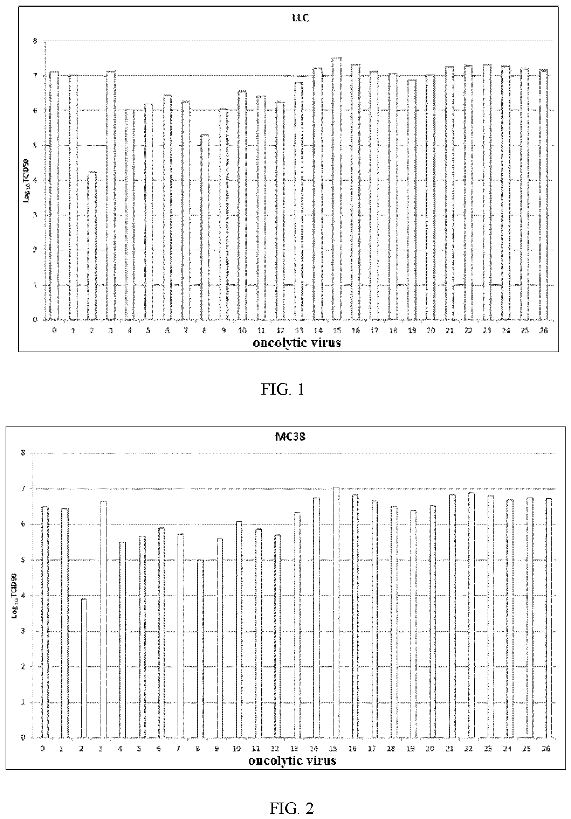

Test results were shown in FIG. 1 to 4. In the figures, 0 on an abscissa represented the wild-type oncolytic virus; 1 to 26 on the abscissa represented the oncolytic viruses prepared in Preparation Examples 1 to 26 respectively. Log₁₀TCID50 on an ordinate represented a value of TCID50 calculated using Karber method. The larger of the value of Log₁₀TCID50, the better of the infective ability of the oncolytic virus to the cell; the smaller of the value of Log₁₀TCID50, the poorer of the infective ability of the oncolytic virus to the cell.

FIG. 1 shown the test result of the infective ability of the oncolytic virus prepared in the present application and the wild-type oncolytic virus to LLC cells.

FIG. 2 shown the test result of the infective ability of the oncolytic virus prepared in the present application and the wild-type oncolytic virus to MC38 cells.

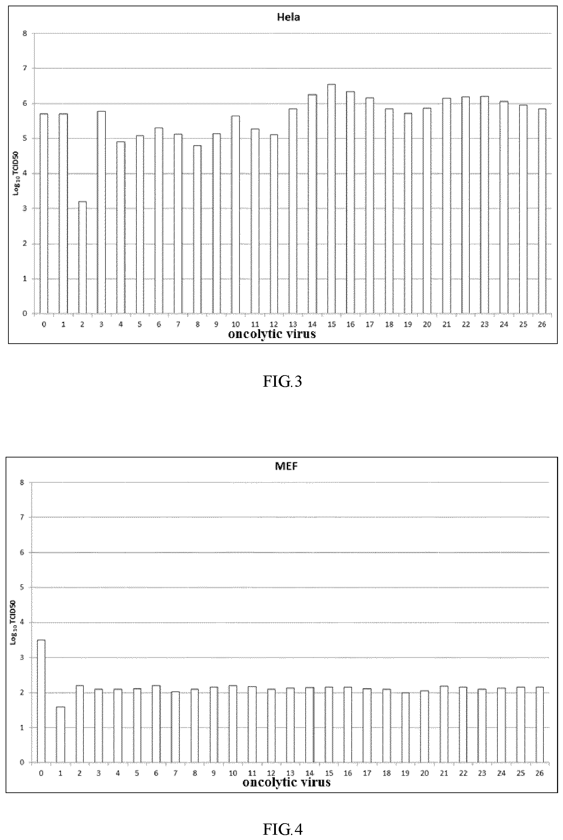

FIG. 3 shown the test result of the infective ability of the oncolytic virus prepared in the present application and the wild-type oncolytic virus to HeLa cells.

FIG. 4 shown the test result of the infective ability of the oncolytic virus prepared in the present application and the wild-type oncolytic virus to MEF cells.

As can be seen from the above figures, the oncolytic viruses prepared in Preparation Examples 1 to 26 of the present application all had better infective ability to LLC cells, MC38 cells and HeLa cells, especially the oncolytic viruses prepared in Preparation Examples 4 to 26. Based on the oncolytic virus prepared in Preparation Example 3, the amino acids on the G protein of the oncolytic virus was further subjected to site-directed mutations, thereby further improving the infective ability of the oncolytic virus to LLC cells, MC38 cells and HeLa cells. At the same time, the oncolytic viruses prepared above had poor infective ability to MEF cells, indicating that the oncolytic virus prepared in the present application can be better used to infect tumor cells, cancer cells and other cells without damaging normal cells, and has a broad application prospect.

### Example 2

In this Example, a killing ability of the oncolytic viruses prepared in Preparation Examples 1 to 26 and the wild-type oncolytic virus in vitro to different cells were respectively detected. A detection method was a MTT detection method, that is, 200 pfu each of the oncolytic viruses prepared in Preparation Examples 1 to 26 and the wild-type oncolytic virus were added into a culture medium of different cells, and a cell activity was detected using the MTT detection method after 24 hours. The cells tested included: LLC cells, MC38 cells, MEF cells, and 4T1 cells (mouse breast cells). Specifically, the detection method included the following steps.
(1) 100 µL of Vero (LLC/MC38/Hela/MEF) cell suspension was added into each well of the 96-well culture plate to make a cell number reach 1×10⁴ cells/well. The 96-well culture plate was cultured for 16 hours under the environmental condition of 37°C and 5% of CO₂.
(2) The oncolytic viruses prepared in preparation Examples were diluted to MOI (multiplicity of infection) of 0.001, 0.01, 0.1, and 1.0 respectively, and each dilution gradient of the oncolytic viruses was inoculated into the 96-well culture plate in step (1). 4 wells for each dilution gradient were inoculated, 100 µL/well, and the 96-well culture plate was cultured for 40 hours under the environmental condition of 37°C and 5% of CO₂.
(3) A cell supernate in the 96-well culture plate in step (2) was removed, and a fresh medium and MTT solution were added to the 96-well culture plate in a volume of 20 µL/well, and the 96-well culture plate was cultured for 4 hours under the environmental condition of 37°C and 5% of CO₂.
(4) The 96-well culture plate was centrifuged for 5 minutes at a room temperature and at 2500 rpm/min, and a supernatant was gently removed using 1mL disposable sterile syringe. Then, DMSO was add into each well of the 96-well culture plate in a volume of 100µL/well, and placed for 10 minutes under the environmental condition of 37°C. An OD value of each well of the 96-well culture plate at a wavelength of 570 nm or 490 nm was measured using a multifunctional microplate reader with shake for 2 minutes.

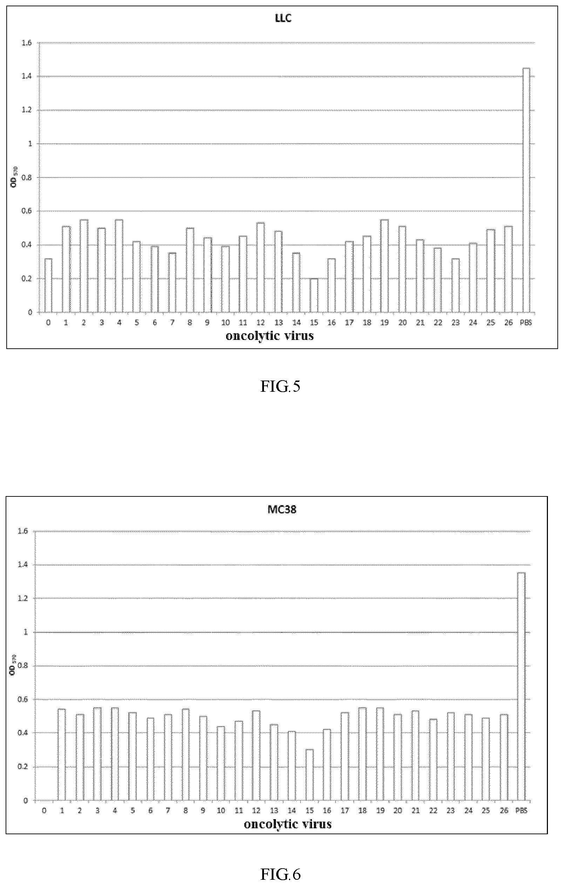

Test results were shown in FIG. 5 to 8. In the figures, 0 on the abscissa represented the wild-type oncolytic virus; 1 to 26 on the abscissa represented the oncolytic viruses prepared in Preparation Examples 1 to 26 respectively. OD₅₇₀ on the ordinate represented the OD value of the cell. The larger of the OD₅₇₀ value, the poorer of the killing ability of the oncolytic virus to the cell; the smaller of the OD₅₇₀ value, the better of the killing ability of the oncolytic virus to the cell.

FIG. 5 shown the test result of the killing ability of the oncolytic virus prepared in the present application and the wild-type oncolytic virus to LLC cells in vitro.

FIG. 6 shown a test result of a killing ability of the oncolytic virus prepared in the present application and the wild-type oncolytic virus to MC38 cells in vitro.

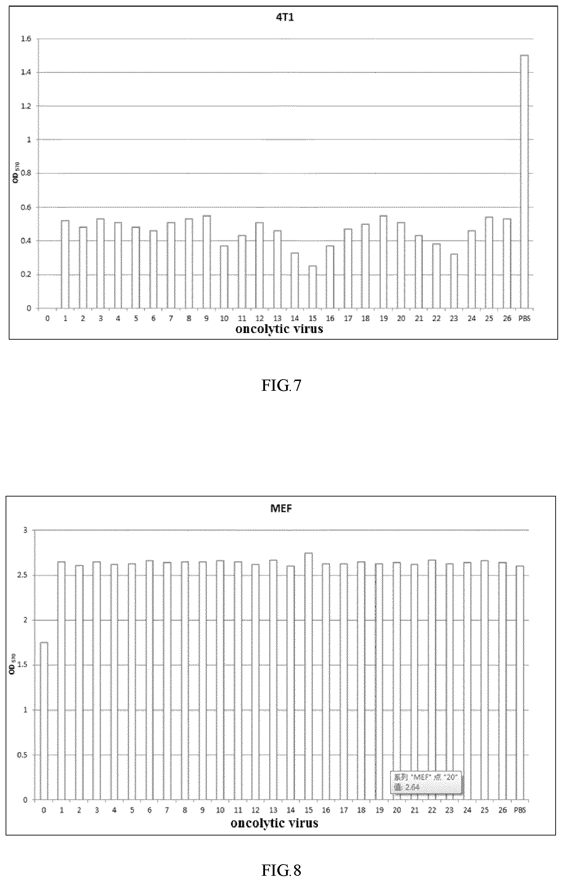

FIG. 7 shown a test result of a killing ability of the oncolytic virus prepared in the present application and the wild-type oncolytic virus to 4T1 cells in vitro.

FIG. 8 shown a test result of a killing ability of the oncolytic virus prepared in the present application and the wild-type oncolytic virus to MEF cells in vitro.

As can be seen from the above figures, the oncolytic viruses prepared in Preparation Examples 1 to 26 of the present application all had better killing ability to LLC cells, MC38 cells and 4T1 cells in vitro, especially the oncolytic viruses prepared in Preparation Examples 4 to 26. Based on the oncolytic virus prepared in Preparation Example 3, the amino acids on the G protein of the oncolytic virus was further subjected to site-directed mutations, thereby further improving the killing ability to LLC cells, MC38 cells and 4T1 cells in vitro. At the same time, the oncolytic viruses prepared above had almost no effect on MEF cells, indicating that the oncolytic virus prepared in the present application can be better used to damage and kill abnormal cells such as tumor cells, cancer cells without damaging normal cells.

Although the wild-type oncolytic virus has better killing ability in vitro to LLC cells, MC38 cells and 4T1 cells, the wild-type oncolytic virus can damage and kill the above cells and also can damage and kill MEF cells to a great extent, limiting the clinical application of the wild-type oncolytic viruses. Therefore, the engineering of the wild-type oncolytic virus in the peresent application not only ensures the safety of the oncolytic virus to normal cells, but also ensures the killing ability of the oncolytic virus to tumors cells and cancer cells, and has a broad clinical application prospect.

### Example 3

In this Example, a elimination of the oncolytic viruses prepared in Preparation Examples 1 to 26 and the wild-type oncolytic virus in different cells were respectively detected. A detection index was an expression of gene IFN-β. The gene IFN-β is a soluble glycoprotein gene produced by cells with extensive anti-viral, anti-tumor and immunomodulatory effects. The expression of gene IFN-β can be used to judge the cell's ability to eliminate the oncolytic virus. When the expression of gene IFN-β is high, the oncolytic virus is easy to eliminate in cells; when the expression of the gene IFN-β is low, the oncolytic virus is not easy to eliminate in cells. The cells tested included: LLC cells, MC38 cells, HeLa cells, and MEF cells. Specifically, the detection method included the following steps.
(1) 100 µL of Vero (LLC/MC38/Hela/MEF) cell and MEF cell suspension was added into each well of the 96-well culture plate to make a cell number reach 1×10⁴ cells/well. The 96-well culture plate was cultured for 16 hours under an environmental condition of 37°C and 5% of CO₂.
(2) The oncolytic viruses prepared in preparation Examples were diluted to MOI (multiplicity of infection) of 0.001, 0.01, 0.1, and 1.0 respectively, and each dilution gradient of the oncolytic viruses was inoculated into the 96-well culture plate in step (1). 4 wells for each dilution gradient were inoculated, 100 µL/well, and the 96-well culture plate was cultured for 40 hours under the environmental condition of 37°C and 5% of CO₂.
(3) Each group of cells cultured and obtained in step (2) were broken, and total RNA was extracted from each cell by using TRIzol (Invitrogen) , and reverse transcribed into cDNA by using a reverse transcription kit of PrimeScript RT Reagent Kit with DNA Eraser (Takara), then dyed with dyestuff of LightCycler 480SYBR Green I Master (Roche), and a Ct value of each gene was detected by using LightCycle 480 quantitative PCR instrument. A relative expression level of target gene IFN-β was calculated by ΔΔ Ct method.

Test results were shown in FIG. 5 to 8. In the figures, 0 on the abscissa represented the wild-type oncolytic virus; 1 to 26 on the abscissa represented the oncolytic viruses prepared in Preparation Examples 1 to 26 respectively. IFN-β level on the ordinate represented the expression of IFN-β gene. The larger of the value of IFN-β level, the easier of the oncolytic virus to eliminate in the cell; the smaller of the value of IFN-β level, the more difficult of the oncolytic virus to eliminate in the cell.

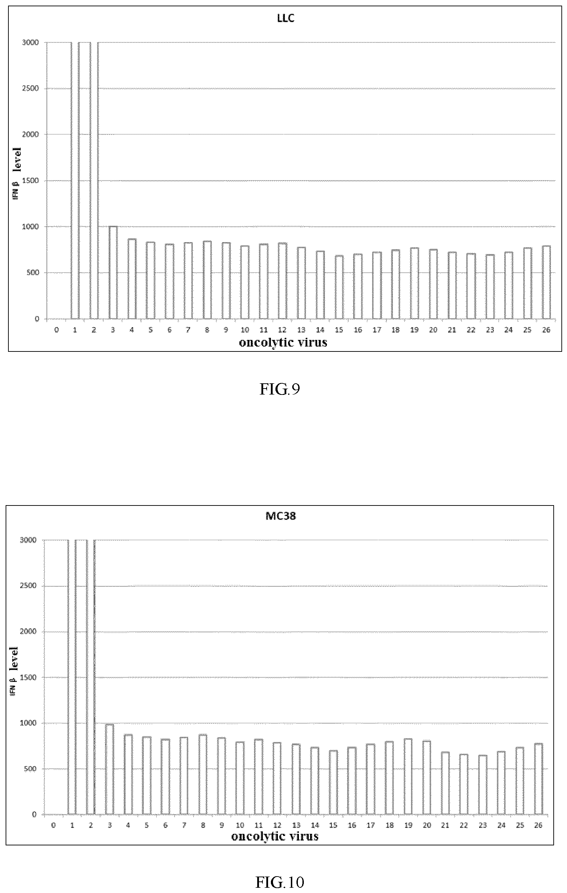

FIG. 9 shown the test result of the elimination of the oncolytic virus prepared in the present application and the wild-type oncolytic virus in LLC cells.

FIG. 10 shown the test result of the elimination of the oncolytic virus prepared in the present application and the wild-type oncolytic virus in MC38 cells.

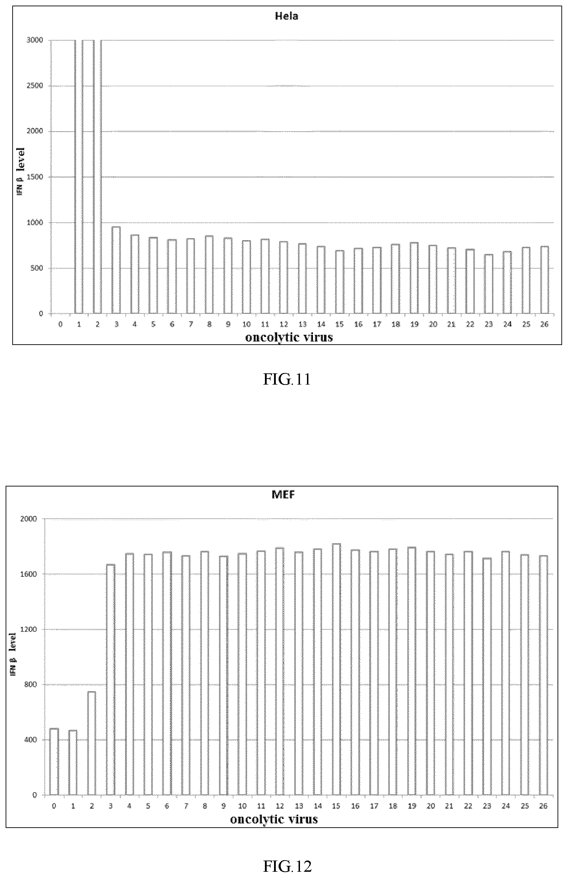

FIG. 11 shown the test result of the elimination of the oncolytic virus prepared in the present application and the wild-type oncolytic virus in HeLa cells.

FIG. 11 shown the test result of the elimination of the oncolytic virus prepared in the present application and the wild-type oncolytic virus in MEF cells.

As can be seen from the above figures, the oncolytic viruses prepared in Preparation Examples 1 to 2 were not easy to eliminate in LLC cells, MC38 cells and HeLa cells. Compared with the oncolytic viruses prepared in Preparation Examples 1 and 2, the oncolytic virus prepared in Preparation Example 3 is easier to eliminate in LLC cells, MC38 cells and HeLa cells. Based on the oncolytic virus prepared in Preparation Example 3, the amino acids on the G protein of the oncolytic viruses prepared in Preparation Examples 4 to 26 were further subjected to site-directed mutations, thereby making the oncolytic virus more difficult to eliminate in LLC cells, MC38 cells and HeLa cells, further ensuring that oncolytic virus can better infect and kill in LLC cells, MC38 cells and Hela cells. At the same time, the oncolytic virus was easier to eliminate in MEF cell, further ensuring the safety of MEF cells, thus improving the safety of the oncolytic virus.

The foregoing detailed description is provided in the form of explanations and examples, and is not intended to limit the scope of the attached claims. At present, various changes in the embodiments listed in the present application are obvious to those skilled in the art, and fall into the scope of the claims and their equivalents.

## Claims

1. An oncolytic virus, **characterized by** comprising an M protein and a G protein, wherein the M protein comprises an amino acid sequence as shown in SEQ ID NO: 1 having amino acid substitutions at positions 51, 221, and 226, and the G protein comprises an amino acid sequence as shown in SEQ ID NO: 2 having at least one amino acid substitution.

2. The oncolytic virus according to claim 1, **characterized in that**, the amino acid substitution of the M protein comprises mutating of methionine at position 51 into arginine (M51R); and/or mutating of valine at position 221 into phenylalanine (V221F); and/or mutating of serine at position 226 into arginine (S226R).

3. The oncolytic virus according to any one of claims 1 to 2, **characterized in that**, the M protein comprises an amino acid sequence shown in SEQ ID NO: 5.

4. The oncolytic virus according to any one of claims 1 to 3, **characterized in that**, the G protein comprises an amino acid sequence shown in SEQ ID NO: 2 having, amino acid substitutions at one or more of the following positions: position 438, position 453, position 471 and position 487.

5. The oncolytic virus according to any one of claims 1 to 3, **characterized in that**, the G protein comprises the amino acid sequence shown in SEQ ID NO: 2 having amino acid substitutions at one or more of the following positions: position 53, position 141, position 172, position 217, position 232, position 331, position 371, position 436, position 438, position 453, position 471 and position 487.

6. The oncolytic virus according to any one of claims 1 to 5, **characterized in that**, the amino acid substitution of the G protein comprises mutating of valine at position 53 into isoleucine (V53I); and/or mutating of alanine at position 141 into valine (A141V); and/or mutating of aspartic acid at position 172 into tyrosine (D172Y); and/or mutating of lysine at position 217 into glutamic acid (K217E); and/or mutating of aspartic acid at position 232 into glycine (D232G); and/or mutating of valine at position 331 into alanine (V331A ); and/or mutating of valine at position 371 into glutamic acid (V371E); and/or mutating of glycine at position 436 into aspartic acid (G436D); and/or mutating of valine at position 438 into serine (T438S); and/or mutating of phenylalanine at position 453 into leucine (F453L); and/or mutating of threonine at position 471 into isoleucine (T471I); and/or mutating of tyrosine at position 487 into histidine (Y487H).

7. The oncolytic virus according to claim 6, **characterized in that**, the amino acid substitution of the G protein is selected from any one of the following groups:
1) the amino acid substitution of the G protein by V53I;
2) the amino acid substitutions of the G protein by V53I and A141 V;
3) the amino acid substitutions of the G protein by V53I, A141V and D172Y;
4) the amino acid substitutions of the G protein by V53I, A141V, D172Y and K217E;
5) the amino acid substitutions of the G protein by V53I, A141V, D172Y, K217E and D232G;
6) the amino acid substitutions of the G protein by V53I, A141V, D172Y, K217E, D232G and V331A;
7) the amino acid substitutions of the G protein by V53I, A141V, D172Y, K217E, D232G, V331A and V371E;
8) the amino acid substitutions of the G protein by V53I, A141V, D172Y, K217E, D232G, V331A, V371E and G436D;
9) the amino acid substitutions of the G protein by V53I, A141V, D172Y, K217E, D232G, V331A, V371E, G436D and T438S;
10) the amino acid substitutions of the G protein by V53I, A141V, D172Y, K217E, D232G, V331A, V371E, G436D, T438S and F453L;
11) the amino acid substitutions of the G protein by V53I, A141V, D172Y, K217E, D232G, V331A, V371E, G436D, T438S, F453L and T471I;
12) the amino acid substitutions of the G protein by V53I, A141V, D172Y, K217E, D232G, V331A, V371E, G436D, T438S, F453L, T471I and Y487H;
13) the amino acid substitutions of the G protein by A141V, D172Y, K217E, D232G, V331A, V371E, G436D, T438S, F453L, T471I and Y487H;
14) the amino acid substitutions of the G protein by D172Y, K217E, D232G, V331A, V371E, G436D, T438S, F453L, T471I and Y487H;
15) the amino acid substitutions of the G protein by K217E, D232G, V331A, V371E, G436D, T438S, F453L, T471I and Y487H;
16) the amino acid substitutions of the G protein by D232G, V331A, V371E, G436D, T438S, F453L, T471I and Y487H;
17) the amino acid substitutions of the G protein by V331A, V371E, G436D, T438S, F453L, T471I and Y487H;
18) the amino acid substitutions of the G protein by V371E, G436D, T438S, F453L, T471I and Y487H;
19) the amino acid substitutions of the G protein by G436D, T438S, F453L, T471I and Y487H;
20) the amino acid substitutions of the G protein by T438S, F453L, T471I and Y487H;
21) the amino acid substitutions of the G protein by F453L, T471I and Y487H;
22) the amino acid substitutions of the G protein by T471I and Y487H; and
23) the amino acid substitution of the G protein by Y487H.

8. The oncolytic virus according to claim 6, **characterized in that**, the amino acid substitutions of the G protein comprise substitution by V53I, A141V, D172Y, K217E, D232G, V331A, V371E, G436D, T438S, F453L, T471I and Y487H.

9. The oncolytic virus according to claims 8, **characterized in that**, the G protein comprises an amino acid sequence as shown in SEQ ID NO: 17.

10. The oncolytic virus according to any one of claims 1 to 9, **characterized in that**, the oncolytic virus comprises a rhabdovirus.

11. The oncolytic virus according to any one of claims 1 to 10, **characterized in that**, the oncolytic virus comprises a vesicular stomatitis virus (VSV).

12. The oncolytic virus according to any one of claims 1 to 11, **characterized in that**, the oncolytic virus comprises an Indiana MuddSummer subtype of the VSV.

13. The oncolytic virus according to any one of claims 1 to 12, **characterized in that**, the oncolytic virus comprises or expresses an exogenous target protein.

14. The oncolytic virus according to any one of claims 1 to 13, **characterized in that**, the oncolytic virus comprises a nucleic acid molecule, and the nucleic acid molecule comprises a nucleic acid sequence encoding the M protein with the amino acid substitution and a nucleic acid sequence encoding the G protein with the amino acid substitution.

15. The oncolytic virus according to claim 14, **characterized in that**, the nucleic acid molecule comprises a nucleic acid sequence encoding the exogenous target protein.

16. The oncolytic virus according to claim 15, **characterized in that**, the nucleic acid sequence encoding the exogenous target protein in the nucleic acid molecule is located between an nucleic acid sequence encoding a L protein and the nucleic acid sequence encoding the G protein with the amino acid substitution.

17. The oncolytic virus according to claim 16, **characterized in that**, the nucleic acid molecule encodes a N protein, the L protein and a P protein of the oncolytic virus.

18. An expression vector of the oncolytic virus capable of producing the oncolytic virus according to any one of claims 1 to 17.

19. A virus production cell capable of producing the oncolytic virus according to any one of claims 1 to 17.

20. A pharmaceutical composition, **characterized by** comprising the oncolytic virus according to any one of claims 1 to 17, and optionally a pharmaceutically acceptable carrier.

21. A method for preparing the oncolytic virus according to any one of claims 1 to 17, the expression vector of the oncolytic virus according to claim 18, the virus production cell according to claim 19, and/or the pharmaceutical composition according to claim 20.

22. Use of the oncolytic virus according to any one of claims 1 to 17, the expression vector of the oncolytic virus according to claim 18, the virus production cell according to claim 19, and/or the pharmaceutical composition according to claim 20 in preparing a medicine for preventing and/or treating a disease and/or a disorder.

23. The use according to claim 22, **characterized in that**, the oncolytic virus, the expression vector of the oncolytic virus, the virus production cell and/or the pharmaceutical composition are used in a method for sustained killing of an abnormally proliferative cell.

24. The use according to claim 23, **characterized in that**, the abnormal proliferative cell is selected from a tumor cell or a cell associated with tumor tissue.

25. The use according to claim 24, **characterized in that**, the tumor cell is a cancer cell or a metastatic cancer cell.

26. The use according to any one of claims 22 to 24, **characterized in that**, the use comprises: a solid tumor and/or a hematological tumor.
